# EUROPEAN PATENT SPECIFICATION

(11) **EP 3 113 698 B1**
(45) Date of publication and mention of the grant of the patent: **28.08.2019**
(21) Application number: 15757720.6
(22) Date of filing: 06.03.2015
(51) Int. Cl.: A61B 17/00

(54) **SINGLE INCISION SPECIMEN RETRIEVAL ASSEMBLY**
EINZELINZISIONSPROBENRÜCKGEWINNUNGSANORDNUNG
ENSEMBLE DE RÉCUPÉRATION D'UN ÉCHANTILLON AVEC INCISION UNIQUE

(30) Priority: 07.03.2014 US 201461949806 P
(43) Date of publication of application: 11.01.2017
(73) Proprietor: Beacon Surgical Instruments, LLC, Dobbs Ferry, NY 10522 (US)
(72) Inventor: RAVIKUMAR, Sundaram, Dobbs Ferry, NY 10522 (US); ALWARD, Henry Allan, Dobbs Ferry, NY 10522 (US)
(74) Representative: Davies, Gregory Mark
(86) International application number: PCT/US2015/019218
(87) International publication number: WO 2015/134888

(56) References cited:
- US-A- 5 312 416
- US-A- 5 647 372
- US-A1- 2002 019 640
- US-A1- 2002 082 516
- US-A1- 2004 006 338
- US-A1- 2010 217 299
- US-A1- 2011 184 433

## Description

### FIELD OF THE INVENTION

The present invention relates to a specimen retrieval assembly.

### BACKGROUND OF THE INVENTION

In general, laparoscopic surgery is a minimally invasive surgical technique, in which surgery is performed through several small incisions rather than the traditional larger incision. This technique relies on the use of endoscopes and long-handled instruments that are introduced into the body through an insertion port, or trocar. As endoscope and instrument technology has improved, the technique has become more and more prevalent and has been adapted to virtually every imaginable procedure. Today, laparoscopic surgery is one of the most common surgical techniques in the United States.

The initial opening in the body tissue to allow passage of surgical instruments to the interior of the body may be a natural passageway of the body, or it can be created by a tissue piercing instrument such as a trocar, or created by a small incision into which a cannula is inserted. Because the housings, instrumentation, and any required punctures or incisions are relatively small in laparoscopic surgery, the surgery is less invasive as compared to conventional surgical procedures in which the surgeon is required to cut open large areas of body tissue. Therefore, minimally invasive surgery or laparoscopic surgery minimizes trauma to the patient and reduces patient recovery time and hospital costs. In addition, in certain surgeries the abdominal walls and abdominal organs are minimally damaged during such a surgical operation, so that complications, recovery time of a patient and pain during the recovery of the patient can all be reduced.

Minimally invasive procedures may be used for partial or total removal of body tissue or organs from the interior of the body, e.g. nephrectomy, cholecystectomy, lobectomy, myomectomy, hysterectomy and other procedures including thoracic, laparoscopic and endoscopic procedures. During such procedures, it is common that a cyst, tumor, or other affected tissue or organ needs to be removed via the access opening in the skin, or through a cannula. Various types of entrapment devices have been disclosed to facilitate this procedure. In many procedures where cancerous tumors are removed, removal of the specimen in an enclosed environment is highly desirable to prevent seeding of cancer cells. Further, in certain types of surgery such as cholecystectomy, an infected or inflamed gallbladder is removed and bile must be contained so as not to contaminate the surgical area.

Many types of laparoscopic surgery involve removal of tissue, and virtually all of these surgeries require the use of a specimen pouch, also called an endoscopic specimen bag or an "endo bag" or a laparoscopic bag or "lap-bag". The bag is inserted into the body via a cannula and trocar, in an incision location where another device has been removed or for example through the belly button or umbilicus, and the bag is deployed within the body cavity to receive the tissue, closed, and then removed through one of the incisions, typically the umbilicus, or in females the vagina if a gynecological surgery.

During the abdominal surgery using an endoscope as described above, when an operator performs an operation of removing tumors or suturing internal organs and the like, the operator inserts various medical instruments into an abdomen, together with the endoscope and surgical instruments, to take pieces of tumors or internal organs, which are removed during the abdominal surgery, out of the abdomen by receiving the pieces of tumors or internal organs in a vinyl bag using a medical instrument referred to as a lap-bag.

Conventional lap-bag mechanisms employed in an abdominal surgery using the trocar as described above are well known and comprise a housing or cannula, a wire spring and a folded vinyl bag. In use, the wire spring is deployed from the housing, the bag (including a pull string if the bag will be sealable and pulled out of the body via the string) is deployed, the bag is placed by the surgeon over the specimen, the specimen placed in the open bag by a second instrument via a different incision or trocar, and if included a pull string is engaged sealing the bag holding the specimen, otherwise graspers or other instruments may position a sealing mechanism such as a ring around the opening of the bag to contain the specimen.

In certain surgical operations such as abdominal surgery, an incision is made in the abdomen sufficient for insertion of 10 mm trocar which forms pneumoperitoneum by putting gas (e.g. CO2 gas) into the abdomen during endoscopic surgery so as to create the space and visibility for the endoscopic surgery, and allows an endoscope, a surgical instrument and a medical instrument, such as an endoloop or lap-bag, to be inserted into the abdomen. The trocar and cannula are used for inserting surgical instruments into the abdomen. Given the diameter of the trocar and the various instruments passing therethrough, the incision must be at least 15-20 mm.

Conventional minimally invasive surgeries for cholecystectomy involve the use of four trocars (access devices). In general, one trocar is inserted in the umbilicus, through which an endoscope is inserted, with two trocars being inserted on the right side of the abdomen for retraction and mobilizing the gallbladder, in order to identify the important structures. The fourth trocar is typically inserted in the midline above the umbilicus.

The aforementioned method has become the standard approach and has withstood twenty years of changes in surgical skill sets, in various groups of surgeons. However, even using a 5 mm trocar still leaves a scar and thus there is a need to reduce scarring and need for even small trocars such as a 5 mm version. Relatively recently, even newer and advantageous techniques for cholecystectomy have been developed that involve either two 5 mm trocars or even only a single trocar or "port", called SILS (single incision laparoscopic surgery). As most conventional specimen retrieval bag devices are 10 mm they in turn require a 10 mm trocar opening and thus the less invasive two 5 mm trocar method of surgery cannot be used. Further, the prediction is that nearly twenty to forty percent of all gallbladder surgeries will be performed using SILS as the opening and incision is larger than the two trocar method, but the number of openings or incisions is reduced. This technology involves inserting a single port inserted through the umbilicus, with all the instruments going into the abdominal cavity through the single port. Retrieving the gallbladder is challenging with this technology, especially if the gallbladder is distended due to inflammation.

In conventional cholecystectomy an endoscope or other surgical instruments cannot be inserted through the trocar into which the lap-bag is inserted such that the number of trocars through which an endoscope or surgical instruments can be inserted are increased. This may cause unnecessary surgical damage of the abdomen, increase of the total operation time required for the abdominal surgery and increase possible complications. Other surgeries are also impeded by this approach in that not all instruments may reside within a single trocar, no matter how large the trocar size. Even a 5 mm trocar leaves a scar and may increase complications, pain and recovery time. Thus, a need exists to reduce the size of the main incision during a laparoscopic surgery, typically the incision in which the majority of the instruments will pass through the body wall into the body cavity during the surgery.

Examples of known specimen removal pouches or bags and applicators are described in US 5647372 and US 2002/0082516.

Further, a need exists to reduce the number of incisions during surgery and therefore reduce the potential areas for complications, infection and scarring. Specifically a need exists for a specimen retrieval device which does not need a trocar. One less trocar is thus required. A need exists for a self-inserting specimen retrieval device.

This and other needs are met by the inventive specimen removal assembly. The number of incisions, namely larger incisions needed for a 10 mm or 20 mm trocar, are reduced. Further, the total number of incisions are reduced and total number of trocars needed are reduced, even smaller diameter trocars. In addition, an operation process for removing the specimen can be simplified by using the inventive specimen retrieval assembly and the time and cost for the surgery can be reduced.

Accordingly, there remains a need in the art for the inventive specimen retrieval device. The present invention provides a solution for these needs and other needs.

The present invention has been made to solve the above problems occurring in the prior art and other needs in regard to surgical instruments and methods of treatment.

### SUMMARY OF THE INVENTION

In one aspect, a surgical specimen retrieval assembly is provided as defined in appended claim 1, which is adapted and configured to be self-inserted and retrieve a specimen via an endoscopic bag.

Further optional features of the invention are defined in the dependent claims.

### DESCRIPTION OF THE DRAWINGS

The above and other advantages of the present invention will become readily apparent with reference to the following detailed description when considered in conjunction with the accompanying drawings which are incorporated in and constitute part of this specification, are included to illustrate and provide a further understanding of the devices of the invention and related methods. Together with the description, the drawings serve to explain the principles of the invention, wherein:
FIGURE 1 is a perspective view illustrating one embodiment of the inventive specimen retrieval device with the specimen retrieval bag retracted within the housing.
FIGURE 2 is a perspective of a cut-away view illustrating one embodiment of the inventive specimen retrieval device with the specimen retrieval bag retracted within the housing.
FIGURE 3 is a perspective view of a cut-away exploded view of a distal end of one embodiment of the inventive specimen retrieval with the specimen retrieval bag retracted within the housing
FIGURE 4 is a perspective view of a cut-away exploded view of a distal end of one embodiment of the inventive specimen retrieval with the specimen retrieval bag retracted within the housing.
FIGURE 5 is a perspective view of one embodiment of the inventive specimen retrieval with the specimen retrieval bag deployed.
FIGURE 6 is a perspective view of a cut-away view of one embodiment of the inventive specimen retrieval from the underside showing the pull ring and not the rod grip, with the specimen retrieval bag deployed .
FIGURE 7 is a perspective view of a cut-away exploded view of a deployed specimen retrieval bag in one embodiment of the inventive specimen retrieval with the tip in an active position.
FIGURE 8 is a perspective view of a cut-away exploded view of a deployed specimen retrieval bag in another embodiment of the inventive specimen retrieval with the tip in a resting position.

### DETAILED DESCRIPTION OF THE INVENTION

Reference will now be made in detail to the present invention, examples of which are illustrated in the accompanying drawings.

In accordance with one aspect of the invention, a surgical instrument assembly is provided having the capacity to be self-inserted within the body, deploy a specimen retrieval bag and retrieve the specimen. In accordance with one aspect, the inventive assembly has a distal end which is capable of being self-inserted without the need for a trocar. The assembly includes a specimen retrieval bag within a housing. Once the assembly is inserted into the patient and the target tissue or organ to be removed from the body cavity is in a position relative to the still retracted bag, the specimen retrieval bag is deployed out of the housing. The deployed specimen retrieval bag is moved to encompass the specimen of tissue or an organ, or the target tissue or organ placed within the deployed and opened bag, and then the specimen retrieval bag is detached, cinched, and separated from the specimen removal device. The specimen retrieval device is removed and the specimen retrieval bag including the specimen is removed through the initial incision or through a larger incision elsewhere in the body. The device may include a loop grip mechanism, thumb grip mechanism, pistol grip mechanism or other mechanism for the surgeon to deploy and detach the specimen retrieval bag.

It should be noted that although the devices of the present invention are advantageous for various laparoscopic surgeries, including cholecystectomy procedures, they can advantageously be applied to retrieve other organs, such as the uterus or fibroids, for example, and to retrieve tissue such as tumors.

A device or assembly in accordance with the invention advantageously helps by reducing the need for a series of trocars used during surgery. Notably, the need for a larger trocar, such as a 10 mm, 15 mm or 20 mm trocar for insertion of all instruments during surgery, is reduced as typically a conventional known specimen retrieval device requires a larger trocar. The inventive device is self-inserting so no trocar is needed. Further, advantageously, the small diameter size of the inventive device minimizes or eliminates scarring, complications and pain to the patient.

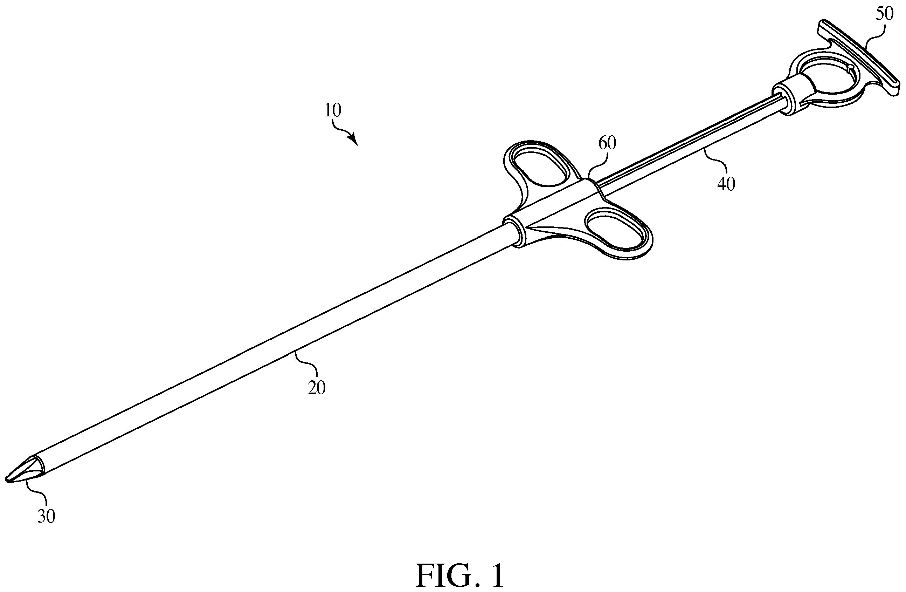

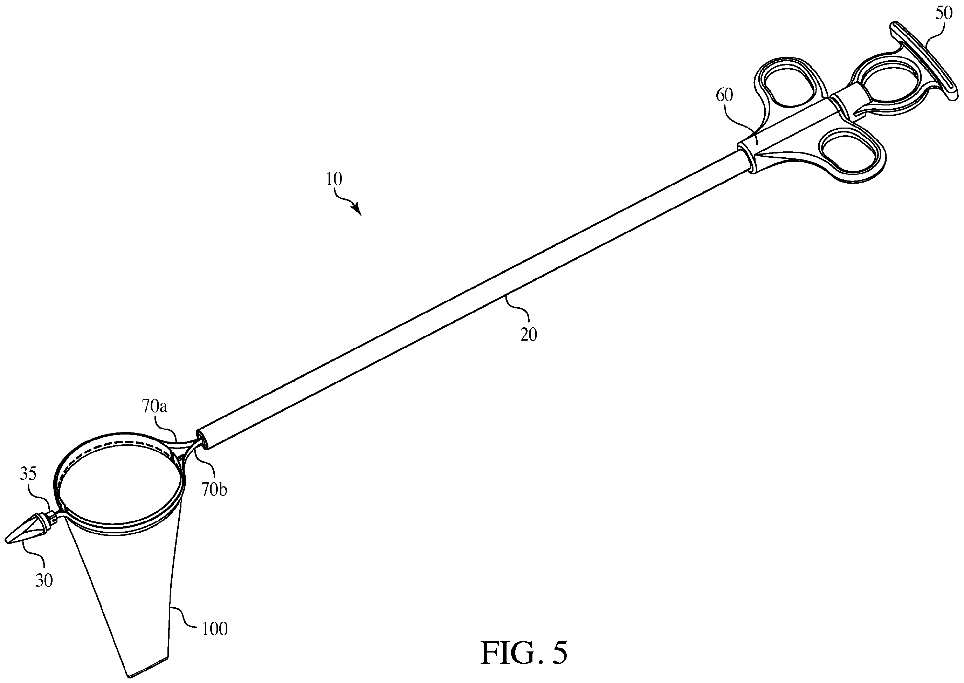

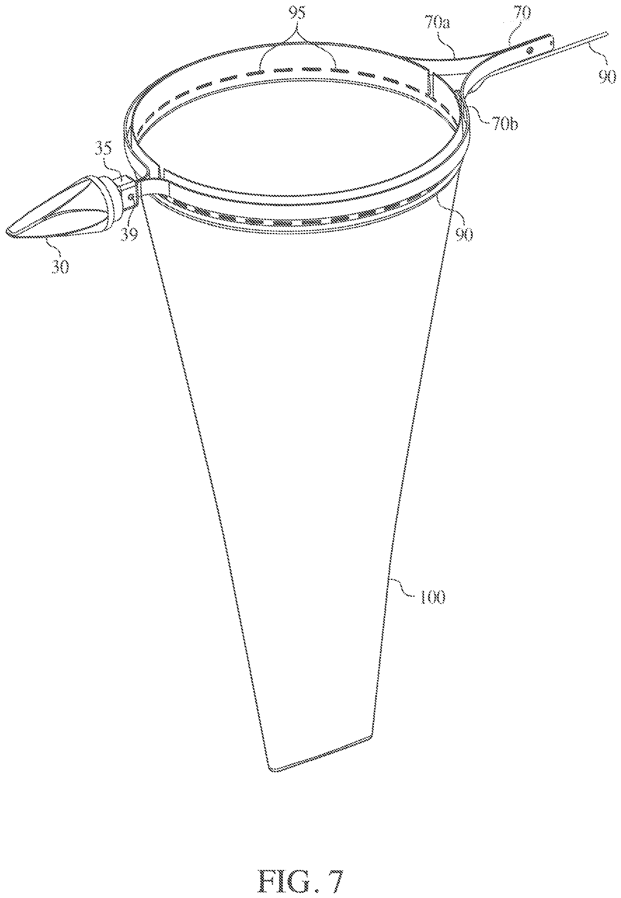

For the purposes of explanation and illustration, and not limitation, in accordance with the invention, an exemplary embodiment of a specimen retrieval assembly in an unactuated position is illustrated in FIGURES 1 through 4 and in an actuated position is illustrated in FIGURES 5 through 8. In accordance with these examples, the specimen retrieval device **10** includes a housing **20** having a diameter in a range of about 1 mm to about 50 mm, with a length of about 200 mm in a range of about 50 mm to about 400 mm of length. The housing **20** may be a tube or cannula. The housing **20** is hollow in which to hold the folded specimen bag (not shown in FIGURE 1, **100**) and a deployment mechanism for the bag. In one embodiment the diameter of the housing **20** is about 7.5 mm, with other embodiments including but not limited to a housing **20** diameter of about 3 mm, 5 mm, 10 mm, 12.5 mm, 15 mm, 16 mm, 20 mm, 25 mm, 30 mm or larger. The diameter of the housing **20** may be related to the diameter of the open bag **(100)** as related to the size of the target tissue or organ. For instance, a pediatric patient may require a smaller diameter such as a 3 mm or 5 mm for the diameter of the housing **20.**

Housing **20** may be comprised of any material compatible to the human body as the distal end of the housing **20** will be placed within the body cavity for deployment of the bag **100.** Such materials include plastics or metals. If a metal, the housing **20** may be comprised of stainless steel and may further be coated with a shrink wrap plastic such as shrinkable polyethylene fiberglass, or polyvinyl chloride of a grade suitable for use in surgical procedures. The housing **20** may be comprised of many known polymers such as polycarbonates or ABS. For cost effectiveness, the housing **20** may be comprised of any plastic capable of sterilization according to regulatory agencies for medical devices.

The dimensions of housing **20** may be a diameter in the range of about 1 mm to about 50 mm, preferably between about 3 mm to about 20 mm. The dimensions of the housing **20** may also be such that the diameter is slightly larger than the maximum diameter of the open specimen retrieval bag **100,** either empty or filled and preferably when filled with the target specimen, such as for example without limitation, if the open diameter of the specimen retrieval bag **100** is 5 mm then the diameter of the housing would be between about 7 mm to about 8 mm.

The housing **10** is hollow and thus includes a channel which houses the folded non-deployed specimen retrieval bag **100** which bag **100** is connected to the deployment mechanism. The deployment mechanism is a rod **40** and a support means **70.** In an unactuated position, the support means **70** is housed within the housing as it is connected to the specimen retrieval bag **100** such that it supports the mouth of the bag **100** when the bag **100** is deployed and open. The rod **40** is partially housed within the housing **20** at the distal end of the rod **40** and proximal end of the housing **20** in the inventive device's **10** unactuated position while the proximal end of the rod **40** extends beyond and out of the proximal end of the housing **20** in the unactuated position. To deploy the specimen retrieval bag **100** the rod **40** is pushed in a downward direction toward the self-inserting distal end tip **30** such that the rod **40** resides fully within the housing **20** with just a portion of the proximal end of the rod **40** extending out of the housing **20.** In the actuated position the specimen retrieval bag **100** is moved from a stowed position within the distal end of the housing **20** to a deployed position by sliding the distal end **45** of the rod **40** from a retracted position within the housing **20** to an extended position at least partially projecting out of the housing **20.** The rod **40** may be connected to a rod grip **50** which the surgeon may use in actuating the inventive device **10,** such as a ring grip, finger grip, thumb grip, pistol grip, or other known grips. In FIGURES 1-6, a ring grip is shown as one embodiment of the rod grip **50** which includes a finger portion such that in use the surgeon can place his or her thumb within the diameter of the ring and two fingers on the lower portion of the flat finger rest or grip portion. A further grip is also shown which assists the surgeon in use on that the grip is connected to the outer diameter of the housing **20**, on a proximal end of the housing **20,** such that the surgeon can grip the housing grip **60** with one hand with gripping the rod grip **50** with the other hand. In use the surgeon would use the housing grip **60**, which in this embodiment has two rings oriented across from one another, to stabilize the housing **20** when using force in a downward direction to deploy the folded and unacted specimen retrieval bag **100** via gripping the rod grip **50.**

Each of the grips **(50, 60)** may be comprised of any material, preferably compatible to the human body though neither will reside within the body cavity. Preferred materials are plastics or metals. If a metal, either grip **(50, 60)** may be comprised of stainless steel and may further be coated with a shrink wrap plastic such as shrinkable polyethylene fiberglass, or polyvinyl chloride of a grade suitable for use in surgical procedures. Either or both of the grips **(50, 60)** may be comprised of many known polymers such as polycarbonates or ABS. For cost effectiveness, the grips **(50, 60)** may be comprised of any plastic capable of sterilization according to regulatory agencies for medical devices.

The deployment mechanism includes a rod **40** and further includes a support means **70** connected to the specimen retrieval bag **100.** The support means may be a spring, a circular metal or polymer piece, a non-circular metal or polymer piece, one arm, two arms, or other known means to support the mouth of a specimen retrieval bag **100** when open. In one embodiment of the invention the support means is two arms **70a, 70b,** which when deployed in an actuated or open position form a circle to support the mouth of a specimen retrieval bag **100** when open. The arms **70a, 70b** comprising the support means **70** may be comprised of any flexible materials which is capable of compression when housed within the housing **20** and capable of forming an open position for the mouth of a specimen retrieval bag **100** when open, once deployed and actuated outside of the distal end of the housing **20.** The support means **70** may be a spring resilient material to give it a tendency to form a circle, oval or other substantially round shape in a fully deployed position. When retracting, the circle or oval collapses under manual force against spring bias to fit within the housing **20.** For instance, the arms **70a, 70b** may be comprised of a flexible metal such as stainless steel, may be a high strength stainless steel alloy, titanium, nitinol, steel spring alloys, metal alloys, plastics, combinations thereof. In general, the support means **70** (such as arms **70a, 70b**) may be comprised of any material that can be stored in a deformed shape, is resilient, and can be returned via compression or other force to an initial or near initial shape.

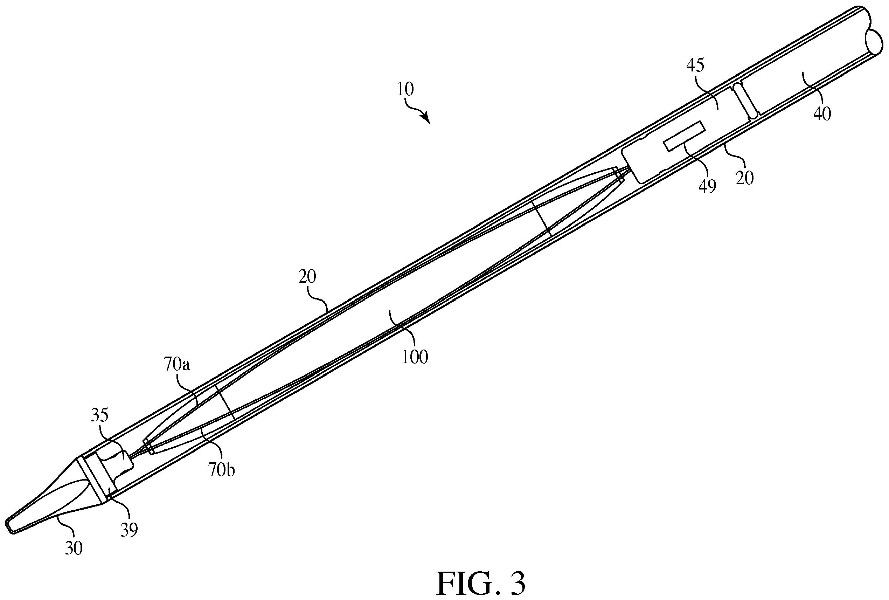

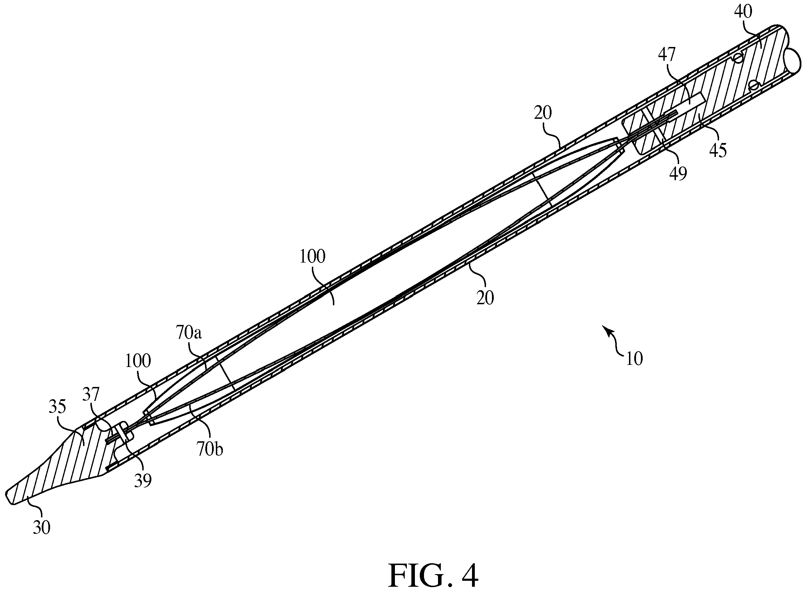

As shown in detail in FIGURES 3 and 4 in one embodiment of the present invention, each of the arms **70a, 70b** is connected on its proximal end to the distal end **45** of the rod **40.** The distal end **45** of the rod **40** includes an aperture **47** in which the proximal end of each of the arms **70a, 70b** may be placed and secured by any known connecting means, such as via a faster, hinge, pin, welding, soldering, crimping, adhesives or another suitable technique, as desired. In one embodiment of the inventive device **10,** the proximal end of each of the arms **70a, 70b** is placed within aperture **47** and a pin **49** secures that portion of the arms **70a, 70b** to the distal end **45** of the rod **40.**

The specimen retrieval bag **100** is detachably connected to the support means **70.** Typically, a specimen retrieval bag **100** has an unsealed mouth portion located at the top end of the specimen retrieval bag **100** and a sealed portion located at the bottom end of the specimen retrieval bag **100.** The specimen retrieval bag **100** may be capable of cinching or closing via a cinching means **90** such as a cinch cord or a string connected to the outer diameter of the specimen retrieval bag **100** at a position below the mouth of the specimen retrieval bag **100.** In some specimen retrieval bags **100,** the string **90** is connected to the specimen retrieval bag **100** via a channel located on the outer circumference of the specimen retrieval bag **100.** Further, the specimen retrieval bag **100** may be capable of detachment from the support means **70** via, for instance, perforations **95** located near mouth and the top of the specimen retrieval bag **100.** Other means to detach the specimen retrieval bag **100** may be employed. Further, other means for cinching, closing or sealing the specimen retrieval bag **100** once the specimen is within it may be employed.

The specimen retrieval bag **100** is not shown in FIGURE 1 as it is folded within a channel in the housing **20.** FIGURES 1-3 show the specimen retrieval device in an un-actuated configuration but since they are cut-away figures the folded and non-deployed specimen retrieval bag **100** is shown. The specimen retrieval bag **100** may be comprised of elastomeric or polymeric material such as but not limited to polyurethane, polyethylene, polypropylene, vinyl, latex, polymers, plastics, or combinations thereof. Any material which is compatible with the body may be used for the specimen retrieval bag **100.** Further, specimen retrieval bag **100** is comprised of a material which is preferably transparent or semi-transparent so that the surgeon can observe the target specimen of tissue or an organ received in the specimen retrieval bag **100** through an endoscope with naked eyes. The specimen retrieval bag **100** may be of a dimension suitable for encompassing the target specimen, such as for instance, having a diameter when open from about 2 mm to about 50 mm.

The specimen retrieval bag **100** in one embodiment of the present invention includes an unsealed mouth and a sealed bottom and may be tapered inward, tapered outward or of a square, rectangle, triangle, oval or circular shape overall. The shape of the specimen retrieval bag **100** may be dependent on the target specimen. In another embodiment of the invention, the specimen retrieval bag **100** may not be capable of cinching and instead remains open throughout the procedure and only partially closes upon force by the body wall when the filled specimen retrieval bag **100** is being pulled out of the body cavity and through the body wall and fascia. In another embodiment of the present invention, the specimen retrieval bag **100,** may not be capable of cinching but may instead be capable of sealing the open mouth after the specimen is placed within the open and deployed specimen retrieval bag **100.**

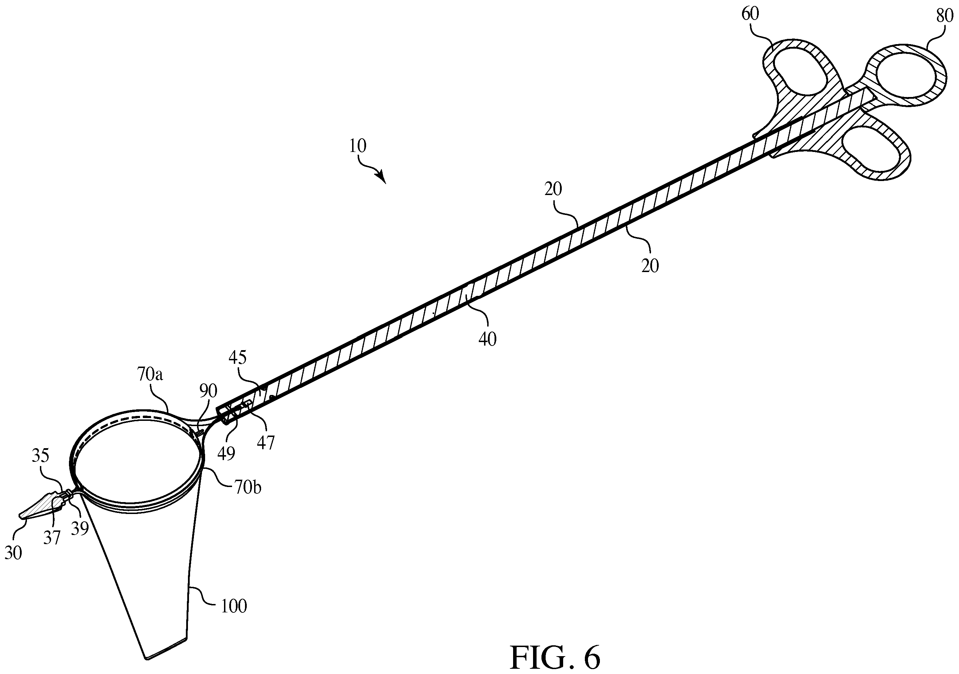

The specimen retrieval bag **100** has a closed lower end and an open upper end forming a mouth. The mouth is detachably connected to the distal ends of the rod such as by a series of perforations **95.** Just below the perforations **95** the specimen retrieval bag **100** includes a cinching or sealing means such as a string **90** which string **90** is sealed within a pouch or channel or sleeve running along the upper end of the specimen retrieval bag **100** configured to cinch or seal the specimen retrieval bag **100** upon detachment. The string **90** is connected at one end to a pull ring **80** (shown in Figure 6) and the string **90** runs the length of the housing **20** through a channel within the rod **40.** Other known detachment means are available for the specimen retrieval bag **100** in addition to perforations, such as scoring or thinning a portion of the material such that it is prone to tear when the string is pulled and the upper end of the specimen retrieval bag **100** is cinched, as well as other means known in the art. The string **90** may also be comprised of silk, cotton or nylon or the like. The string **90** may be replaced by other known means to cinch the specimen retrieval bag **100** such as, but not limited to, cords, nylons, plastics, and the like.

The inventive specimen retrieval device **10** further includes a self-inserting distal end tip **30.** The distal end tip **30** is configured to be self-inserting and has a non-blunt shape. The distal end tip **30** in FIGURES 1-8 is shown as one embodiment in the shape of a cone. In other embodiments of the present invention the distal end tip **30** may be curved, hooked, pointed, bull-nosed, combinations thereof, or any other non-blunt shape which has some sharpened form to be self-inserting within skin during use. The distal end tip should be configured such that it is capable of forming an incision on the fascia of a patient, preferably a human being, with the application of force. In one embodiment the distal end tip **30** may be sharp enough that no incision is needed and it punctures the fascia of the patient itself, such as for instance, without limitation, a needle shape. In other embodiments the distal end tip **30** may be inserted within a defect in the skin surface, such as a prior incision which may or may not be of a smaller diameter than the diameter of the housing **20.** In all instances, the distal end tip **30** is self-inserting in that it does not require a port or trocar. However, the use of a trocar or port is not excluded.

In use, the specimen retrieval device **10** is positioned outside of the body directly over or in the vicinity of the tissue or organ to be retrieved. Force is applied such that the distal end tip **30** penetrates the fascia of the patient. No trocar or port access is needed to insert the specimen retrieval device **10.** In some instances the specimen retrieval device **10** may be inserted into a defect or prior incision area. In other instances a trocar may be removed, for instance having a diameter or 5 mm, and the inventive specimen retrieval device **10** inserted which has an outer diameter of 7.5 mm so that the specimen retrieval device **10** is self-inserting without need for a trocar. Other diameters of the specimen retrieval device **10** may be employed with other defect widths or other trocar diameter sizes. Once within the body at the target location the specimen retrieval bag **100** is deployed such that it unfolds and the mouth opens via the supporting means **70.**

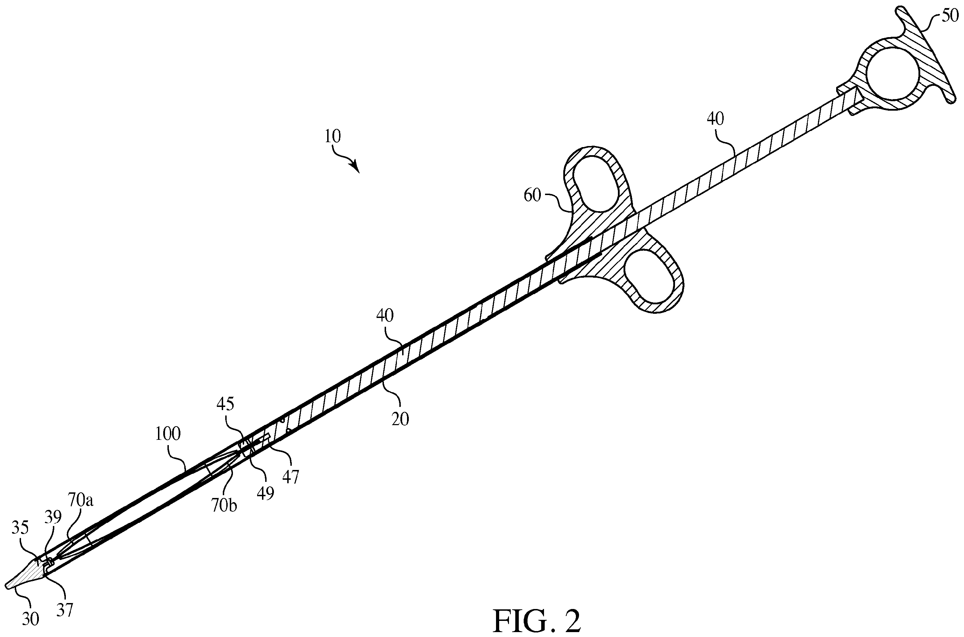

The distal end tip **30** is connected to the support means via any known connecting means. In one embodiment of the present invention, as shown in FIGURES 2-7, the distal end tip **30** is connected to the distal end of each of the arms **70a, 70b.** The distal end tip **30** includes an aperture **37** within the proximal end **35** of the distal end tip **30.** The distal end of each of the arms **70a, 70b** may be placed and secured to the proximal end **35** of the distal end tip **30** by any known connecting means, such as via a faster, hinge, pin, welding, soldering, crimping, adhesives or another suitable technique, as desired. In one embodiment of the inventive device **10,** the proximal end of each of the arms **70a, 70b** is placed within aperture **37** and a pin **39** secures that portion of the arms **70a, 70b** to the proximal end **35** of the distal end tip **30.**

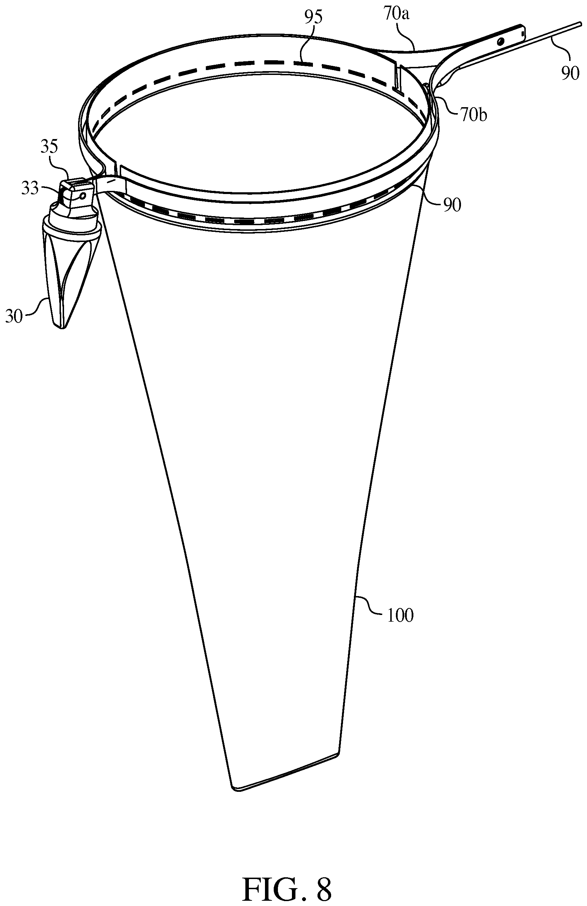

In a further embodiment of the present invention, the distal end tip **30** is connected to the support means via a hinge such the distal end tip **30** may move in a downward direction once within the body cavity. This embodiment may be employed within a body cavity where space is tight so as to reduce any unintentional nicks of non-target tissue or organs by the distal end tip **30.** For instance, the specimen retrieval device **10** may be employed in a pediatric patient or in a body location of an adult patient which even when insufflated, has many non-target tissue and organs within close proximity to the distal end tip **30** when the device **10** is placed within the body cavity. In that instance, the distal end tip **30** may be moved in a downward position once it has been inserted within the body wall and either prior to or after deployment of the specimen retrieval bag. In such an embodiment of the present invention, as shown in FIGURE 8, the distal end tip **30** is connected to the distal end of each of the arms **70a, 70b** via a hinge **33** such that the distal end tip **30** is movable.

A method of use of the inventive specimen retrieval device **10** includes the initial step of inserting, through sufficient force exerted by the surgeon, the distal end tip **30** into the fascia of a patient, through the body wall and into the body cavity. Such insertion is visualized from within the insufflated body cavity via a camera endoscope already placed within the body cavity. No trocar or access port is needed for insertion of the specimen retrieval device **10.** Indeed, a trocar may actually be removed and the specimen retrieval device **10** is self-inserted within the existing defect or incision point. The specimen retrieval device **10** is in a non-actuated state at this point in the method.

Once the target tissue or organ is identified within the body cavity, the surgeon will place the distal end of the housing **20** in the vicinity therein and deploy the specimen retrieval bag **100.** Deployment occurs when the rod **40** is advanced longitudinally distally through the housing **20** by the operator's force, such as pushing, of the rod grip **50,** while maintaining the distal end of the housing **20** within the body cavity by gripping the housing grip **60** to stabilize the housing's **20** location. This movement of the rod **40** pushes the support means **70** and the specimen retrieval bag **100** beyond the distal end and out of housing **20** and, therefore, the support means **70** resiliently pops open to its substantially round configuration to thereby open the mouth of the specimen retrieval bag **100.** The specimen retrieval device **10** is thus actuated and the specimen retrieval bag **100** unfolds as the specimen retrieval bag **100** is moved from a stowed position to a deployed position by sliding the rod **40** from a retracted position, partially within the housing on its distal end and partially outside the housing at the proximal end of the rod **40,** to an extended position of the distal end of the rod **40** at least partially projecting out of the housing **20.** Further manipulation of the deployed specimen retrieval bag **100** may occur via surgical instruments such as graspers inserted through the same or a separate incision, cannula or trocar.

The specimen retrieval bag **100** is moved so as to encompass the target tissue or organ within the specimen retrieval bag **100** or the target tissue or organ is placed via graspers or other surgical instruments within the open and deployed specimen retrieval bag **100.** At this point the filled specimen retrieval bag **100** is detached from the main body of the specimen retrieval device **10** and cinched. A pull ring **80,** which is connected to the string **90** of the specimen retrieval bag **100,** may be grasped and pulled thereby causing the specimen retrieval bag **100** to cinch via the proximal movement of the string and in turn detach from the support means **70** due to the movement of the string and force on the perforations **95** thereby detaching while cinching. At this point the specimen retrieval bag **100,** including the target specimen, has a closed mouth and is detached from the support means **70** but still connected to the device **10** solely via the string **90.**

In one embodiment of the inventive specimen retrieval device **10,** the string **90** proximately located and connected to the pull ring **80** is severed via a razor (not shown) connected and located within an aperture on a lower portion of the rod grip **50.** The string **90** is inserted into the aperture and cut by the razor to allow for complete detachment of the specimen retrieval bag **100** from the specimen retrieval device **10.** The fully detached specimen retrieval bag **100** is thus capable of removal separate from the specimen retrieval device **10.** Other known detachment means are available such as scissors, knives, razors, non-blunt surfaces and may be used to sever the string (or any other cinching or sealing means).

After the specimen retrieval bag **100** has been detached from the support means **70,** the support means **70** is retracted, or pulled, so as to compress and withdraw back into housing **20,** whereupon support means **70** refolds back into its pre-deployed relatively straight configuration to permit removal of the specimen retrieval device **10** from the patient's body. The detached string **90** remains partially outside of the patient's body upon removal of the specimen retrieval device **10.** The string **90** is used to remove the specimen retrieval bag **100** via force applied, such as pulling, and thus the specimen retrieval bag **100** including the target specimen is removed via the incision formed by the distal end tip **30.** In another embodiment of the inventive method, the portion of the string located outside the patient's body may not be used to remove the specimen retrieval bag **100** and instead the portion of the string **90** located within the patient's body may be used to remove the specimen retrieval bag **40** via a trocar or other incision located on the patient's body such as through the umbilicus. In such an embodiment the portion of the string **90** located within the patient's body is manipulated via another surgical instrument such as a grasper or vacuum instrument.

Another aspect of the present invention is a surgical kit which includes the specimen retrieval device **10.** The kit is stored in a sterile sealed package. The kit may include a trocar, a scissors device, a grasper device, and the specimen retrieval device **10.** The kit may optionally include a cauterizing device such as a bi-polar device. Other optional devices may be included. In one embodiment of the inventive kit all components are single use only and disposable.

Advantages of the inventive specimen retrieval device **10** include the configuration such that it is self-inserting, which reduces the need for a trocar. Accordingly the incision location is smaller and may cause less surgical damage to the fascia, reduce the total operation time required for the procedure and reduce possible complications. Further, the surgical process for removing a specimen can be simplified by using the inventive specimen retrieval device **10** and the time and cost for the surgery can be reduced.

Thus, the specimen retrieval device **10** may reduce complications, surgical processes, time and cost.

Many possible combinations could be within the specimen retrieval device, the methods of use, and the kit or system of the present disclosure.

Although the exemplary embodiments of the present invention have been described, it is understood that the present invention should not be limited to these exemplary embodiments but various changes and modifications can be made by one ordinary skilled in the art.

The invention has been described in terms of embodiments thereof, but is more broadly applicable as will be understood by those skilled in the art. The scope of the invention is only limited by the following claims.

## Claims

1. A surgical specimen retrieval assembly (10), comprising:
a hollow housing (20);
a deployment rod (40) that has a proximal end out of the housing and a distal end (45) within the housing (20), which distal end (45) is configured to slide from a retracted position within the hollow of the hollow housing (20) to an extended position to at least partially project out of the hollow housing (20);
a flexible support ring (70) having two portions (70a, 70b) movable between a folded position that fits within the hollow of the housing (20) and an unfolded position clear of the hollow of the housing (20), the flexible support ring (70) being connected to the deployment rod (40) so as to move in unison with movement of the deployment rod (40) from the retracted position to the extended position, the two portions of the flexible support ring (70) being movable from the folded position to the unfolded position under spring bias as the flexible support ring (70) clears the hollow of the housing (20) and the distal end (45) of the deployment rod enters the extended position from the retracted position;
a specimen bag (100) removably connected to the flexible support ring (70) and movable from a stowed position within the hollow of the hollow housing (20) to a deployed position fully clear of the hollow housing (20) as the distal end (45) of the deployment rod (40) moves from the retracted position into the extended position, said specimen bag (100) having a mouth, the mouth being configured to be selectively opened and closed;
**characterised by**
a self-inserting distal end tip (30) connected to a distal end of the flexible support ring (70), said distal end tip (30) having a region that is non-blunt shaped to sufficiently insert into skin.

2. The surgical specimen retrieval assembly of claim 1, wherein the self- inserting distal end tip (30) is shaped in a form selected from the group consisting of a cone, bull-nose, curved, hooked, triangle and combinations therein.

3. The surgical specimen retrieval assembly of claim 1, further comprising cinching means (90) connected to the specimen bag (100) for closing the mouth of the specimen bag, the cinching means (90) including a drawstring encircling the specimen bag (100) through a channel on an outer layer of the specimen bag (100) and pulling means (80) for pulling the drawstring, the pulling means (80) being housed within a finger and thumb grip (50) connected to the proximal end of the deployment rod (40).

4. The surgical specimen retrieval assembly of claim 3, wherein said pulling means (80) includes a ring.

5. The surgical specimen retrieval assembly of claim 1, wherein said housing (20) includes a finger grip (60) on an outer diameter of the housing wherein the finger grip (60) includes a first finger ring and a second finger ring each ring oriented across from each other.

6. The surgical specimen retrieval assembly of claim 1, wherein the specimen bag (100) is transparent or translucent.

7. The surgical specimen retrieval assembly of claim 1, wherein the specimen bag (100) has perforations (95) that sever in response to sufficient manual force to detach the specimen bag (100) from the flexible support ring (70).

## Patentansprüche

1. Chirurgische Probenentnahmeanordnung (10), die Folgendes umfasst:
ein hohles Gehäuse (20);
eine Ausrichtstange (40), die ein proximales Ende außerhalb des Gehäuses und ein distales Ende (45) innerhalb des Gehäuses (20) aufweist, wobei das distale Ende (45) so konfiguriert ist, dass es aus einer eingefahrenen Position innerhalb des Hohlraums des hohlen Gehäuses (20) in eine ausgefahrene Position gleiten kann, um mindestens teilweise aus dem hohlen Gehäuse (20) herauszustehen;
einen flexiblen Stützring (70) mit zwei Abschnitten (70a, 70b), die zwischen einer eingeklappten Position, die in den Hohlraum des Gehäuses (20) passt, und einer ausgeklappten Position, die sich außerhalb des Hohlraums des Gehäuses (20) befindet, bewegbar sind, wobei der flexible Stützring (70) mit der Ausrichtstange (40) verbunden ist, um sich im Einklang mit der Bewegung der Ausrichtstange (40) aus der eingeklappten Position in die ausgeklappte Position zu bewegen, die beiden Abschnitte des flexiblen Stützrings (70) unter Federvorspannung aus der eingeklappten Position in die ausgeklappte Position bewegbar sind, während der flexible Stützring (70) die Aussparung des Gehäuses (20) freigibt und das distale Ende (45) der Ausrichtstange aus der eingefahrenen Position in die ausgefahrene Position eintritt;
einen Probenbeutel (100), der lösbar mit dem flexiblen Stützring (70) verbunden und von einer eingefahrenen Position innerhalb des Hohlraums des Hohlgehäuses (20) in eine ausgefahrene Position bewegbar ist, die vollständig aus dem Hohlgehäuse (20) herausgefahren ist, während sich das distale Ende (45) der Ausrichtstange (40) aus der eingefahrenen Position in die ausgefahrene Position bewegt, wobei der Probenbeutel (100) eine Öffnung aufweist, wobei die Öffnung konfiguriert ist, um selektiv geöffnet und geschlossen zu werden;
**gekennzeichnet durch** eine selbsteinführende distale Endspitze (30), die mit einem distalen Ende des flexiblen Stützrings (70) verbunden ist, wobei die distale Endspitze (30) einen Bereich aufweist, der nicht stumpf geformt ist, um ausreichend in die Haut einzudringen.

2. Chirurgische Probenentnahmeanordnung nach Anspruch 1, wobei die selbsteinführende distale Endspitze (30) in einer Form geformt ist, die aus der Gruppe ausgewählt ist, die aus einem Kegel, einer abgerundeten Kante (bull-nose), einer gekrümmten Nase, einem Haken, einem Dreieck und Kombinationen derselben besteht.

3. Chirurgische Probenentnahmeanordnung nach Anspruch 1, ferner umfassend ein mit dem Probenbeutel (100) verbundenes Verschlussmittel (90) zum Schließen der Öffnung des Probenbeutels, wobei das Verschlussmittel (90) einen den Probenbeutel (100) umgebenden Kordelzug durch einen Kanal auf einer Außenschicht des Probenbeutels (100) und ein Zugmittel (80) zum Ziehen des Kordels einschließt, wobei das Zugmittel (80) in einem Finger und Daumengriff (50) untergebracht ist, der mit dem proximalen Ende des Ausrichtstabes (40) verbunden ist.

4. Chirurgische Probenentnahmeanordnung nach Anspruch 3, wobei das Zugmittel (80) einen Ring einschließt.

5. Chirurgische Probenentnahmeanordnung nach Anspruch 1, wobei das Gehäuse (20) einen Fingergriff (60) an einem Außendurchmesser des Gehäuses einschließt, wobei der Fingergriff (60) einen ersten Fingerring und einen zweiten Fingerring einschließt, wobei jeder Ring gegeneinander ausgerichtet ist.

6. Chirurgische Probenentnahmeanordnung nach Anspruch 1, wobei der Probenbeutel (100) transparent oder lichtdurchlässig ist.

7. Chirurgische Probenentnahmeanordnung nach Anspruch 1, wobei der Probenbeutel (100) Perforationen (95) aufweist, die sich als Reaktion auf eine hinreichende manuelle Kraft durchtrennen, um den Probenbeutel (100) vom flexiblen Stützring (70) zu lösen.

## Revendications

1. Ensemble de récupération d'échantillon chirurgical (10), comprenant :
un logement creux (20) ;
une tige de déploiement (40) qui a une extrémité proximale à l'extérieur du logement et une extrémité distale (45) à l'intérieur du logement (20), laquelle extrémité distale (45) est configurée pour coulisser d'une position rétractée à l'intérieur du creux du logement creux (20) à une position étendue pour faire saillie au moins partiellement à l'extérieur du logement creux (20) ;
une bague de support flexible (70) ayant deux parties (70a, 70b) mobiles entre une position pliée qui s'ajuste à l'intérieur du creux du logement (20) et une position dépliée libre du creux du logement (20), la bague de support flexible (70) étant reliée à la tige de déploiement (40) de façon à se déplacer de concert avec un mouvement de la tige de déploiement (40) de la position rétractée à la position étendue, les deux parties de la bague de support flexible (70) étant mobiles de la position pliée à la position dépliée sous une sollicitation de ressort à mesure que la bague de support flexible (70) libère le creux du logement (20) et que l'extrémité distale (45) de la tige de déploiement entre dans la position étendue à partir de la position rétractée ;
un sac à échantillon (100) relié de façon amovible à la bague de support flexible (70) et mobile d'une position rangée à l'intérieur du creux du logement creux (20) à une position déployée complètement libre du logement creux (20) à mesure que l'extrémité distale (45) de la tige de déploiement (40) se déplace de la position rétractée dans la position étendue, ledit sac à échantillon (100) ayant une embouchure, l'embouchure étant configurée pour être sélectivement ouverte et fermée ;
**caractérisé par** une pointe d'extrémité distale à auto-insertion (30) reliée à une extrémité distale de la bague de support flexible (70), ladite pointe d'extrémité distale (30) ayant une région qui est de forme non émoussée pour s'insérer suffisamment dans la peau.

2. Ensemble de récupération d'échantillon chirurgical selon la revendication 1, dans lequel la pointe d'extrémité distale à auto-insertion (30) est profilée sous une forme choisie dans le groupe constitué d'un cône, d'un nez arrondi, incurvée, accrochée, d'un triangle et de combinaisons en son sein.

3. Ensemble de récupération d'échantillon chirurgical selon la revendication 1, comprenant en outre un moyen de serrage (90) relié au sac à échantillon (100) pour fermer l'embouchure du sac à échantillon, le moyen de serrage (90) incluant un cordon entourant le sac à échantillon (100) par l'intermédiaire d'un canal sur une couche externe du sac à échantillon (100) et un moyen de traction (80) pour tirer le cordon, le moyen de traction (80) étant logé au sein d'une prise pour doigt et pouce (50) reliée à l'extrémité proximale de la tige de déploiement (40).

4. Ensemble de récupération d'échantillon chirurgical selon la revendication 3, dans lequel ledit moyen de traction (80) inclut une bague.

5. Ensemble de récupération d'échantillon chirurgical selon la revendication 1, dans lequel ledit logement (20) inclut une prise pour doigt (60) sur un diamètre externe du logement dans lequel la prise pour doigt (60) inclut une première bague de doigt et une deuxième bague de doigt chaque bague étant orientée l'une à travers l'autre.

6. Ensemble de récupération d'échantillon chirurgical selon la revendication 1, dans lequel le sac à échantillon (100) est transparent ou translucide.

7. Ensemble de récupération d'échantillon chirurgical selon la revendication 1, dans lequel le sac à échantillon (100) a des perforations (95) qui se séparent en réponse à une force manuelle suffisante pour détacher le sac à échantillon (100) de la bague de support flexible (70).
